# EUROPEAN PATENT APPLICATION

(11) **EP 2 492 831 A1**
(43) Date of publication of application: **29.08.2012**
(21) Application number: 11154080.3
(22) Date of filing: 10.02.2011
(51) Int. Cl.: G06F 19/00, A61B 5/145

(54) **Biomedical device with near field communication (NFC) function and method thereof for user identification, biomedical data measurement, biomedical data upload/download, biomedical data management, and remote medical care**

(71) Applicant: Pensiero Medical Electronics Corp., Xizhi District New Taipei (CN)
(72) Inventor: Chen, Chun-Yu, Zhonghe City, Taipei County (TW); Yang, Chia-Wen, Zhonghe City, Taipei County (TW)
(74) Representative: Becker Kurig Straus

(57) **Abstract**

The present invention relates to a biomedical device with near field communication (NFC) function and a method thereof for user identification, biomedical data measurement, biomedical data upload/download, biomedical data management, and remote medical care, the biomedical device comprises: a measuring unit, a environment sensing unit, a data transforming unit, a micro control unit, a memory unit, a smart card unit, a display unit, and a near field communication unit, wherein by way of near field communication unit, the biomedical device is able to communicate with a portable device and a user individual information and the relative biomedical data thereof can be transmitted to the portable device, such that multi men are allowed for using the identical biomedical device and the situation about misrecording and misjudging of data would not be occurred; moreover, through the portable device, the biomedical data can be uploaded to a cloud server and a user history data and an optimized setting data for personalization biomedical device can be downloaded from the cloud server.

## Description

### BACKGROUND OF THE INVENTION

### 1. Technical Field

The present invention relates to a biomedical device, and more particularly, to a biomedical device with near field communication (NFC) function and a method thereof for user identification, biomedical data measurement, biomedical data upload/download, biomedical data management, and remote medical care.

### 2. Description of Related Art

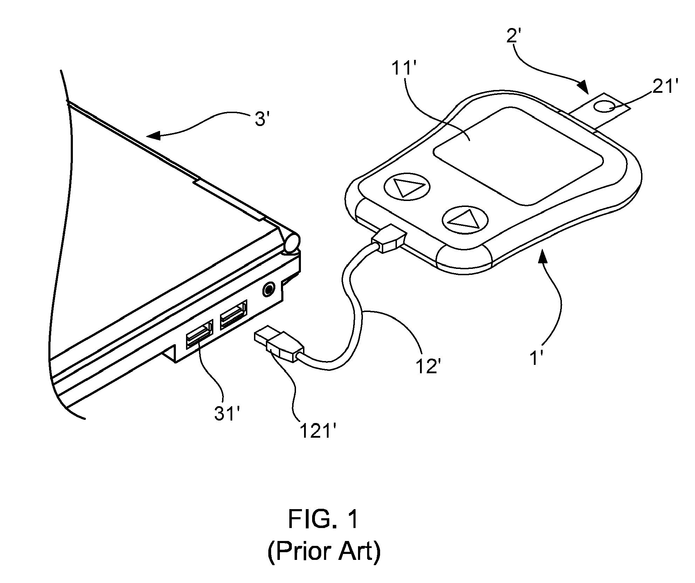

Currently, there are various biomedical device widely used in human life, such as blood glucose meter, body weight scale, blood pressure gauge, electrocardiograph (ECG) machine, and pulse oximeter. Please refer to FIG. 1, which illustrates a stereo view of a conventional blood glucose meter and a computer. As shown in FIG. 1, a user is able to insert a strip 2' into a conventional blood glucose meter 1' and drop a blood in a reaction district 21' of the strip 2', and then the blood glucose meter 1' would calculate the concentration of the blood glucose; furthermore, after calculating, the value of the blood glucose concentration is shown in a display 11' of the blood glucose meter 1'.

Generally, the blood glucose meter 1' merely shows the value of the blood glucose concentration in the display 11', and the user need to record and trace himself daily blood glucose concentration value by using others electronic device, for example, a computer. As shown in FIG. 1, a connection cable 12' having an USB connector 121' is added to the blood glucose meter 1', wherein the USB connector 121' is adopted for connecting to an USB slot 31' of a computer 3'; So that, the user is able to upload his own daily blood glucose concentration value to the computer 3' through an application software installed in the computer 3'. Thus, by way of the connection and communication between the blood glucose meter 1' and the computer 3', it is helpful for the user to clearly record and trace the daily blood glucose concentration thereof.

Although it is convenient for the user to record and trace his own daily blood glucose concentration value by connecting and communicating the blood glucose meter 1' with the computer 3', it is still inconvenient for the user to record the blood glucose concentration value himself by using the computer 3'. On the other hand, with the development of the science and technology, the relatedness between 3C products, biotechnologies and medical services are continually increased, and generally, that is integration for cross domain technologies, such as images, database and internet network. Therefore, it is aware of that, for the needs of the user, it is urgent to integrate the biomedical device with the internet network and the database.

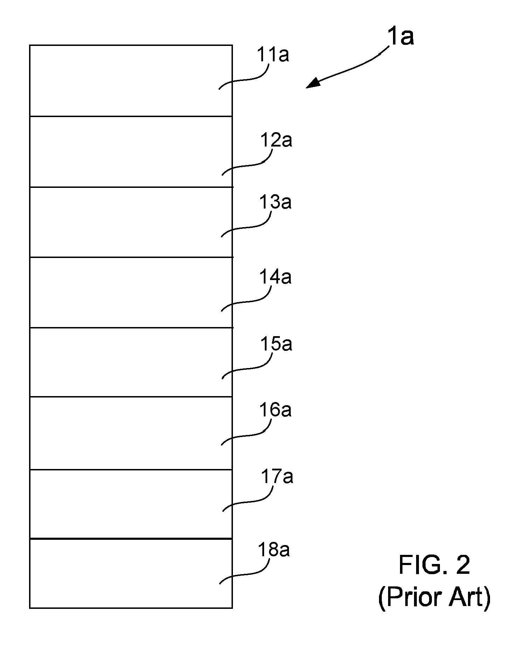

Accordingly, a biomedical measurement device having long-distance transmission function is disclosed in a R.O.C. patent with patent number I292311. Please refer to FIG. 2, which illustrates a block framework view of a biomedical device with long-distance transmission function disclosed by the R.O.C. patent with the patent number I292311. As shown in FIG. 2, the biomedical device 1a with long-distance transmission function includes: a measuring unit 11a, a storing unit 12a, a detecting unit 13a, an internet server unit 14a, a mail server unit 15a, a web server unit 16a, an IP selection unit 17a, and a communication unit 18a.

For the biomedical measurement device 1a described above, wherein the measuring unit 11a is used for measuring a biomedical data, the storing unit 12a is able to store the biomedical data, and the internet server unit 14a provides a internet connecting service, so as to facilitate the biomedical device 1a capable of uploading the biomedical data to a remote server through internet. The mail server unit 15a provides an Email sending service; thus, the user can mail the biomedical data to the remote server by way of Email. The web server unit 16a provides a web interface service; through the web interface service, the monitoring staff may inquire the relative information of the biomedical data stored in the storing unit 12a.

Continuously refer to FIG. 2, the detecting unit 13a is used for determining whether the measured biomedical data exceeds the range of a standard value; when the biomedical data exceeds the standard value, the detecting unit 13a immediately outputs a warning message, and sends out the warning message through the mail server unit 15a. In addition, the communication unit 18a is able to communicate with others biomedical devices for receiving a variety of biomedical data measured by the others biomedical devices.

Thus, through above descriptions, it knows that the biomedical device 1a disclosed by the R.O.C. patent I292311 includes multi functionalities, in which, not only measuring the biomedical data, the biomedical device 1a can also uploads the biomedical data to the remote server; However, the biomedical device 1a proposed by the R.O.C. patent I292311 still has the shortcomings and drawbacks as follows:

1. The biomedical device 1a is a personal using device, in general, the biomedical device 1a immediately uploads the biomedical data to the remote server through the internet server unit 14a after the user finishes the measuring process of personal biomedical data; according to the above description, it can knows that the remote server will make a misrecording in the biomedical data once a second user use the biomedical device 1a to measure himself biomedical data.

2. The biomedical device 1a includes the internet server unit 14a, the mail server unit 15a, the web server unit 16a, the IP selection unit 17a, and the communication unit 18a, such that the production cost of the biomedical device 1a can not be reduced and the product price thereof is expensive.

Moreover, since the smart phone is widely used, how to make the present biomedical device communicate with the smart phone, to manage the user individual information and the relative biomedical data by the smart phone, and to upload these data to the remote server have been became an important issue and widely studied. Accordingly, in order to facilitate an identical biomedical device be allowed for multi men to use and manage the user individual information and the relative biomedical data by the smart phone, the inventors of the present application have made great efforts to make inventive research thereon and eventually provided a biomedical device with near field communication function (NFC) and a method thereof for user identification, biomedical data measurement, biomedical data upload/download, biomedical data management, and remote medical care.

### BRIEF SUMMARY OF THE INVENTION

The first objective of the present invention is to provide a biomedical device with near field communication (NFC) function, in which, a near field communication unit is utilized to communicate with a portable device, and a smart card unit is used for storing a user individual information and the relative biomedical data, therefore, by way of identifying the user individual information, multi men are allowed for using the identical biomedical device and the situation about misrecording and misjudging of data would not be occurred.

The second objective of the present invention is to provide a biomedical device with near field communication (NFC) function, in which, a near field communication unit is utilized to communicate with a portable device, therefore users are able to download a user history data and an optimized setting data for personalization biomedical device thereof from a cloud server through a communication module of the portable device, furthermore, by way of near field communication, the user history data and the optimized setting data for personalization biomedical device can be transmitted to the biomedical device, such that the biomedical device is able to complete a basic parameter setting procedure according to the optimized setting data for personalization biomedical device.

The third objective of the present invention is to provide a biomedical device with near field communication (NFC) function, in which, a near field communication unit is utilized to communicate with a portable device, so that, through the near field communication unit, the user individual biomedical data and information can be transmitted to the portable device, so as to unload the user individual biomedical data and information to a cloud server by using the portable device, therefore the cloud server is able to immediately execute a medical care procedure.

The fourth objective of the present invention is to provide a method used by a biomedical device with near field communication (NFC) function for user identification, biomedical data measurement, biomedical data upload/download, biomedical data management, and remote medical care, through the method, a communication and data exchange procedure between the biomedical device and a portable device can be finished, moreover, the biomedical data, the user individual information, the GPS information, the time information, and the environment temperature/humidity information are able to be uploaded to a cloud server through a communication module of the portable device, so that the cloud server is able to judge the data and then to immediately execute a medical care procedure.

Accordingly, to achieve the abovementioned first, second and third objectives, the inventor proposes a biomedical device with near field communication (NFC) function, which is capable of communicating with a portable device having at least one near field communication module and a smart card, and able to exchange data with portable device by way of near field communication, the biomedical device comprising:
a measuring unit, used for connecting an object under test, so as to measure an analog biomedical signal of the object under test;
an environment sensing unit, capable of simultaneously detecting a temperature/humidity parameter of the external environment when the measuring unit measures the analog biomedical signal;
a data transforming unit, coupled to the measuring unit for receiving the analog biomedical signal, so as to transform the analog biomedical signal to a digital biomedical signal;
a micro control unit, coupled to the environment sensing unit and the data transforming unit, and adopted for simultaneously receiving the digital biomedical signal and the temperature/humidity parameter in order to sort and process the digital biomedical signal and the temperature/humidity parameter to a biomedical data;
a memory unit, coupled to the micro control unit for being one storage media;
a smart card unit, coupled to the micro control unit for being one storage media;
a display unit, coupled to the micro control unit and capable of receiving the biomedical data outputted by the micro control unit, so as to show a biomedical value; and
a near field communication (NFC) unit, coupled to the smart card unit, wherein the near field communication unit is used for accessing the smart card unit, moreover, the near field communication unit is able to transmit the biomedical data stored in the smart card unit to the near field communication module of the portable device;
wherein the user must put the near field communication unit near to the near field communication module of the portable device before the object under test is connected to the measuring unit, such that the near field communication module can communicate with the near field communication unit, and the near field communication module can transmit a user individual information, a GPS information, a time information, an optimized setting data for personalization biomedical device, and an environment temperature/humidity information stored in the smart card to the near field communication unit 18; furthermore, the near field communication unit storing the user individual information, the GPS information, the time information, the optimized setting data for personalization biomedical device, and the environment temperature/humidity information in the smart card unit, so as to complete a user identification procedure and a parameter setting procedure.

Moreover, to achieve the abovementioned fourth objective, the inventor proposes a method used by a biomedical device with near field communication (NFC) function for user identification, biomedical data measurement, biomedical data upload/download, biomedical data management, and remote medical care, the method comprises the steps of:

(1) putting a biomedical device with near field communication (NFC) function near to a portable device having at least one near field communication module and a smart card;

(2) determining whether starting a communication and data exchange procedure between the biomedical device and the portable device, if yes, proceeding to step (3), otherwise, proceeding to step (21);

(3) a near field communication unit communicating with the near field communication module of the portable device;

(4) downloading a user individual information, a GPS information, a time information, an optimized setting data for personalization biomedical device, and an environment temperature/humidity information from a cloud server by a communication module of the portable device;

(5) storing the user individual information, the GPS information, the time information, the optimized setting data for personalization biomedical device, and the environment temperature/humidity information in a smart card;

(6) accessing the smart card by the near field communication module;

(7) the near field communication module transmitting the user individual information, the GPS information, the time information, the optimized setting data for personalization biomedical device, and the environment temperature/humidity information to the near field communication unit;

(8) the near field communication unit storing the user individual information, the GPS information, the time information, the optimized setting data for personalization biomedical device, and the environment temperature/humidity information in a smart card unit;

(9) the biomedical device completing a parameter setting procedure according to the user individual information and the optimized setting data for personalization biomedical device;

(10) determining whether downloading a user history measurement data from the cloud server by using the portable device, if yes, proceeding to step (11), otherwise, proceeding to step (12);

(11) downloading the user history measurement data from the cloud server through the communication module;

(12) determining whether starting to use the biomedical device for measuring an analog biomedical signal of an object under test, if yes, proceeding to step (13), otherwise, proceeding to step (19);

(13) connecting the object under test to a measuring unit of the biomedical device;

(14) measuring the analog biomedical signal of the object under test by the measuring unit, and detecting a temperature/humidity parameter of an external environment by an environment sensing unit;

(15) a data transforming unit receiving the analog biomedical signal and transforming the analog biomedical signal to a digital biomedical signal;

(16) a micro control unit receiving the digital biomedical signal and the temperature/humidity parameter;

(17) the micro control unit sorting and processing the digital biomedical signal and the temperature/humidity parameter to a biomedical data;

(18) storing the biomedical data in the smart card unit by the micro control unit;

(19) determining whether transmitting the biomedical data stored in the smart card unit to the portable device, if yes, proceeding to step (20), otherwise, proceeding to step (25);

(20) the near field communication unit transmitting the biomedical data to the near field communication module;

(21) storing the biomedical data in the smart card by the near field communication module;

(22) determining whether uploading the biomedical data to the cloud server, if yes, proceeding to step (23), otherwise, proceeding to step (25);

(23) uploading the biomedical data, the user individual information, the GPS information, the time information, and the environment temperature/humidity information to the cloud server by using the communication module of the portable device;

(24) the cloud server immediately executing a medical care procedure;

(25) determining whether ending the communication and data exchange procedure between the biomedical device and the portable device, if yes, proceeding to step (26), otherwise, proceeding to step (3);

(26) determining whether ending using the biomedical device to measure the analog biomedical signal of the object under test, if yes, proceeding to step (27), otherwise, proceeding to step (13); and

(27) making the biomedical device with near field communication function to be distant from the portable device.

### BRIEF DESCRIPTION OF THE SEVERAL VIEWS OF THE DRAWINGS

The invention as well as a preferred mode of use and advantages thereof will be best understood by referring to the following detailed description of an illustrative embodiment in conjunction with the accompanying drawings, wherein:
FIG. 1 is a stereo view of a conventional blood glucose meter and a computer;
FIG. 2 is a block framework view of a biomedical device with long-distance transmission function disclosed by a R.O.C. patent with patent number I292311;
FIG. 3 is the block framework view of a biomedical device with near field communication (NFC) function according to the present invention;
FIG. 4 is the stereo view of the biomedical device with NFC function and a portable device having a near field communication module and a smart card;
FIG. 5 is a second block framework view of the biomedical device with NFC function according to the present invention;
FIG. 6 is the second block framework view of the portable device;
FIG. 7 is the stereo view of a body fat and blood pressure meter and the portable device;
FIG. 8 is the stereo view of an electrocardiography meter and the portable device;
FIGs. 9A to 9F are the flow chart of a method used by a biomedical device with near field communication (NFC) function for user identification, biomedical data measurement, biomedical data upload/download, biomedical data management, and remote medical care according to the present invention;
FIG. 10 is a detailed flow chart of step (S05);
FIG. 11 is the detailed flow chart of step (S06);
FIG. 12 is the detailed flow chart of step (S08);
FIG. 13 is the detailed flow chart of step (S18);
FIG. 14 is the detailed flow chart of step (S21); and
FIG. 15A and FIG. 15B are the detailed flow chart of step (S24).

### DETAILED DESCRIPTION OF THE INVENTION

To more clearly describe a biomedical device with near field communication (NFC) function and a method thereof for user identification, biomedical data measurement, biomedical data upload/download, biomedical data management, and remote medical care according to the present invention, embodiments of the present invention will be described in detail with reference to the attached drawings hereinafter.

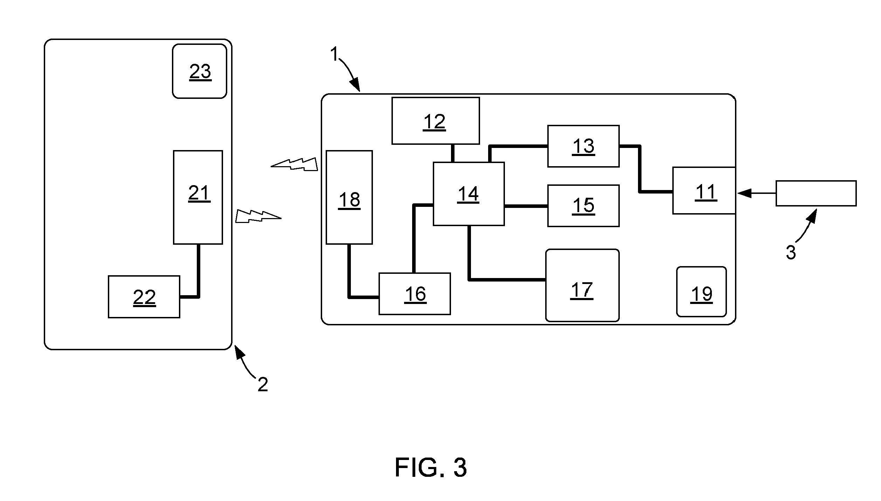

Please refer to FIG. 3, which illustrates the block framework view of a biomedical device with near field communication (NFC) function according to the present invention. As shown in FIG. 3, the biomedical device 1 with NFC function is capable of communicating with a portable device 2 having a near field communication module 21 and a smart card 22, and able to exchange data with portable device 2 by way of near field communication; Generally, the biomedical device can be a blood glucose meter, a body fat machine, a sphygmomanometer, a pulse oximeter, and an electrocardiograph (ECG) machine; besides, the portable device 2 can be a smart phone, a PDA, a portable game console, a digital photo frame, an iPAD, a notebook, and a tablet PC; however, herein the blood glucose meter is as the main embodiment of the biomedical medical device 1 of the present invention and the smart phone is as the portable device 2. The biomedical medical device 1 includes: a measuring unit 11, an environment sensing unit 12, a data transforming unit 13, a micro control unit 14, a memory unit 15, a smart card unit 16, a display unit 17, a near field communication (NFC) unit 18, a power unit 19.

The power unit 19 is used for providing power to the measuring unit 11, the environment sensing unit 12, the data transforming unit 13, the micro control unit 14, the display unit 17, and the near field communication unit 18. The measuring unit 11 is used for connecting an object under test 3, so as to measure an analog biomedical signal of the object under test 3, wherein the object under test 3 is a blood glucose strip. The environment sensing unit 12 can simultaneously detect a temperature/humidity parameter of the external environment when the measuring unit 11 measures the analog biomedical signal. The data transforming unit 13 is coupled to the measuring unit 11 for receiving the analog biomedical signal, so as to transform the analog biomedical signal to a digital biomedical signal. In addition, the micro control unit 14 is coupled to the environment sensing unit 12 and the data transforming unit 13 and adopted for simultaneously receiving the digital biomedical signal and the temperature/humidity parameter, so as to sort and process the digital biomedical signal and the temperature/humidity parameter to a biomedical data.

Continuously refer to FIG. 3, the memory unit 15 and the smart card unit 16 are simultaneously coupled to the micro control unit 14 for being one storage media. The display unit 17 is coupled to the micro control unit 14 and capable of receiving the biomedical data outputted by the micro control unit 14, so as to show a biomedical value. The near field communication unit 18 is coupled to the smart card unit 16, wherein the near field communication unit 18 is used for accessing the smart card unit 16 transmitting the biomedical data stored in the smart card unit 16 to the near field communication module 21 of the portable device 2.

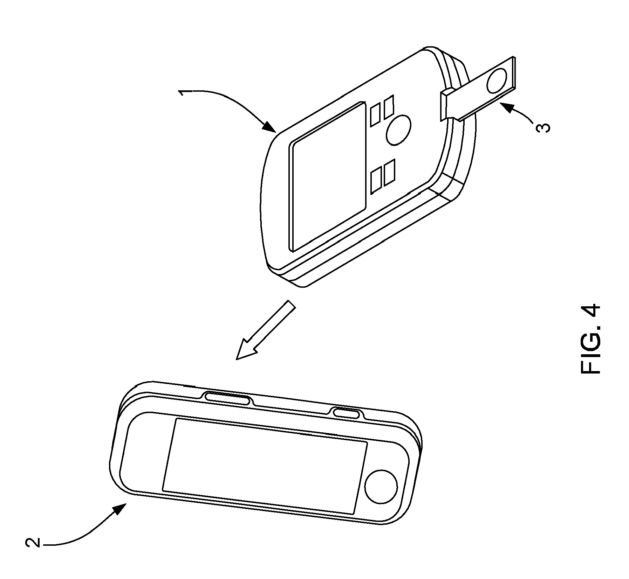

Referring to FIG. 3 again, and simultaneously refer to FIG. 4, which illustrates the stereo view of the biomedical device with NFC function and the portable device with the near field communication module and the smart card. As shown in FIG. 4, when a user wants to use the biomedical device 1, the user must put the near field communication unit 18 near to the near field communication module 21 of the portable device 2 before the object under test 3 is connected to the measuring unit 11, such that the near field communication module 21 can communicate with the near field communication unit 18, and the near field communication module 21 is able to transmit a user individual information, a GPS information, a time information, an optimized setting data for personalization biomedical device, and an environment temperature/humidity information stored in the smart card 22 to the near field communication unit 18; furthermore, the near field communication unit 18 would store the user individual information, the GPS information, the time information, the optimized setting data for personalization biomedical device, and the environment temperature/humidity information in the smart card unit 16, so that the biomedical device 1 is able to complete a user identification procedure and a parameter setting procedure according to the user individual information and the optimized setting data for personalization biomedical device. Therefore, after the user identification procedure and the parameter setting procedure are finished, the user can further connect the object under test 3 to the measuring unit 11 for measuring the analog biomedical signal, or transmit the biomedical data store in the smart card unit 16 to the near field communication module 21 through the near field communication unit 18, so as to store the biomedical data in the smart card 22 of the portable device 2.

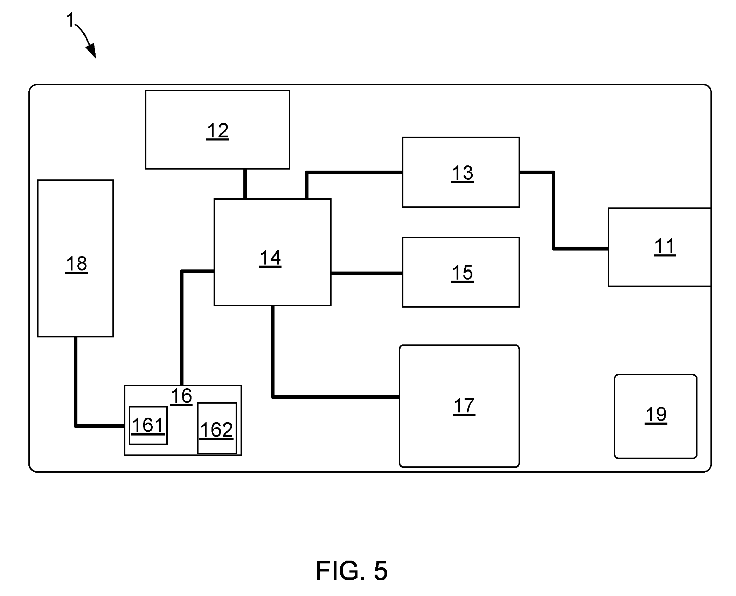

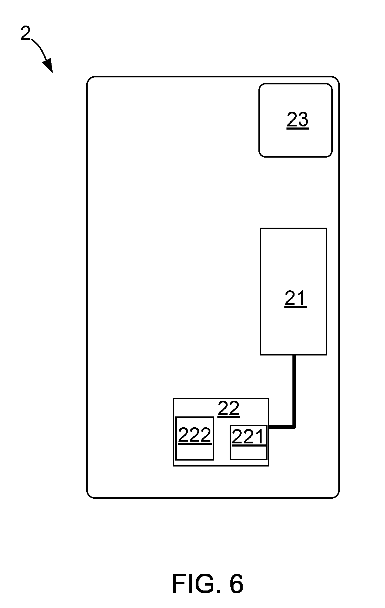

Moreover, please refer to FIG. 5 and FIG. 6, which illustrate a second block framework view of the biomedical device with NFC function and the portable device, respectively. As shown in FIG. 5, in the biomedical device 1 of the present invention, the smart card unit 16 further includes a first micro processor unit 161 and a first micro memory unit 162, wherein first micro processor unit 161 is coupled to the micro control unit 14 and the near field communication unit 18 for receiving the biomedical data, the user individual information, the GPS information, the time information, the optimized setting data for personalization biomedical device, and the environment temperature/humidity information. The first micro memory unit 162 is coupled to the first micro processor unit 161, wherein the first micro processor unit 161 stores the user individual information, the GPS information, the time information, the optimized setting data for personalization biomedical device, and the environment temperature/humidity information in the first micro memory unit 162 after the biomedical data is received.

In addition, as shown in FIG. 6, the smart card 22 further includes a second micro processor unit 221 and a second micro memory unit 222, wherein the second micro processor unit 221 is coupled to the near field communication module 21, and the second micro memory unit 222 is coupled to the second micro processor unit 221. In the portable device 2, the second micro processor unit 221 is used to access the second memory unit 222, so as to read out the user individual information, the GPS information, the time information, the optimized setting data for personalization biomedical device, and the environment temperature/humidity information stored in the second micro memory unit 222, and store the biomedical data in the second micro memory unit 222.

Referring to FIGs. 3-6 again, after the object under test 3 is connected to the measuring unit 11 and the analog biomedical signal is measured by the measuring unit 11, the analog biomedical signal is eventually processed to the biomedical data by the micro control unit 14. It must to be noted that the biomedical data would be further stored in the memory unit 15 via the micro control unit 14; moreover, through the first micro processor unit 161, the biomedical data would also be stored in the first memory unit 162 of the smart card unit 16. Furthermore, after the biomedical data is stored, the user can directly transmit the biomedical data to the portable device 2, or transmit the biomedical data to the portable device 2 through the near field communication unit 18 when next time using the biomedical device 1.

Moreover, in order to immediately provide medical care to the user (patient), when the near field communication module 21 of the portable device 2 receives the biomedical data transmitted by the near field communication unit 18, the second micro processor unit 221 would store the biomedical data in the second micro memory unit 222, and then the biomedical data, the user individual information, the GPS information, the time information, and the environment temperature/humidity information would be further uploaded to a cloud server with a database by way of using a communication module 23 of the portable device 2. Therefore, the cloud server records all received data in the database, and to determine whether the value of the biomedical data is in the range of a standard value. Once the cloud server found that the present physical condition of the user (patient) is must to be concerned, the cloud server would immediately notify and demand a nearby medical unit immediately go to the real location of the user, so as to provide an immediate medical care for the user. Besides, before the user use the biomedical device 1 to measure the biomedical data thereof, the user is able to download a history data recorded in the database from the cloud server for being a comparison data.

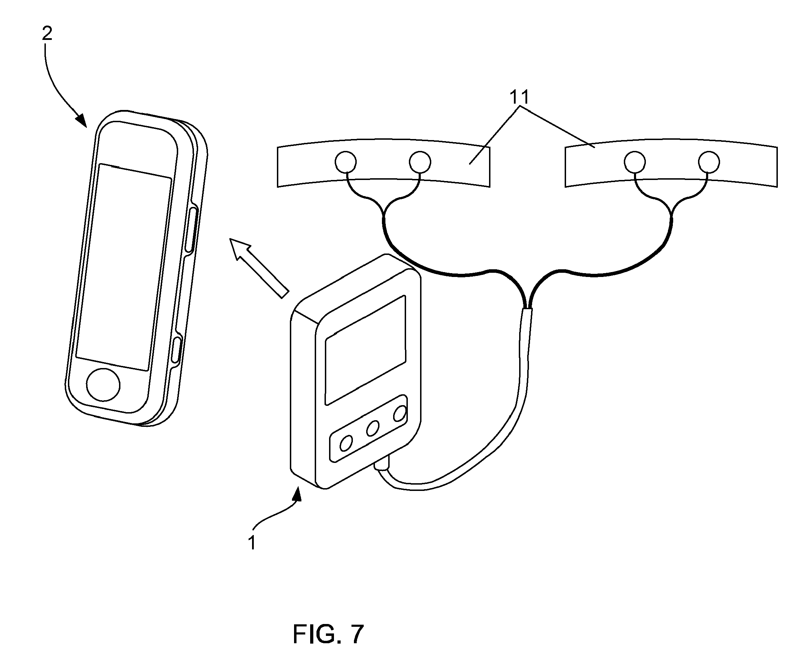

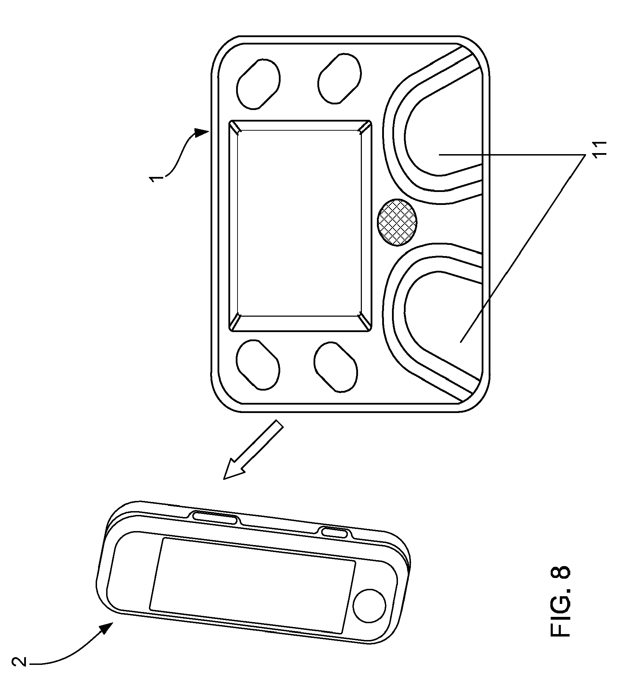

In the above descriptions, the blood glucose meter is used as the embodiment for introducing the framework and the functionalities of the biomedical device with NFC function of the present invention, however, the framework of the biomedical device with NFC function of the present invention can also be applied in the body fat machine, the sphygmomanometer, the pulse oximeter, and the electrocardiograph (ECG) machine. Please refer to FIG. 7, which illustrates the stereo view of the body fat and blood pressure meter and the portable device. as shown in FIG. 7, the measuring unit 11 is two ring electrodes which are able to measure the body fat and the blood pressure of the user by way of surrounding the left wrist and the right wrist of the user, respectively; Moreover, please refer to FIG. 8, which illustrates the stereo view of an electrocardiography meter and the portable device; as shown in FIG. 8, the biomedical device 1 of the present invention also can be the electrocardiography meter, in which the measuring unit 11 is two electrodes. The user can place two fingers thereof on the two electrodes, such that the electrocardiography meter is able to measure and plot an electrocardiography of the user. So that, the same to the above-mentioned biomedical device 1 using the blood glucose meter as the embodiment thereof, when the body fat and blood pressure meter and the electrocardiography meter measure and calculate the body fat value, the blood pressure value and the electrocardiography of the man body (i.e. the biomedical data of the object under test), the body fat value, the blood pressure value and the electrocardiography are stored in the smart card unit 16 and further transmitted to the portable device 2, after that these biomedical data can be upload to the cloud server by way of using the communication module 23 of the portable device 2; and then, through the cloud server, the biomedical data can be managed and the user would get the immediately medical care.

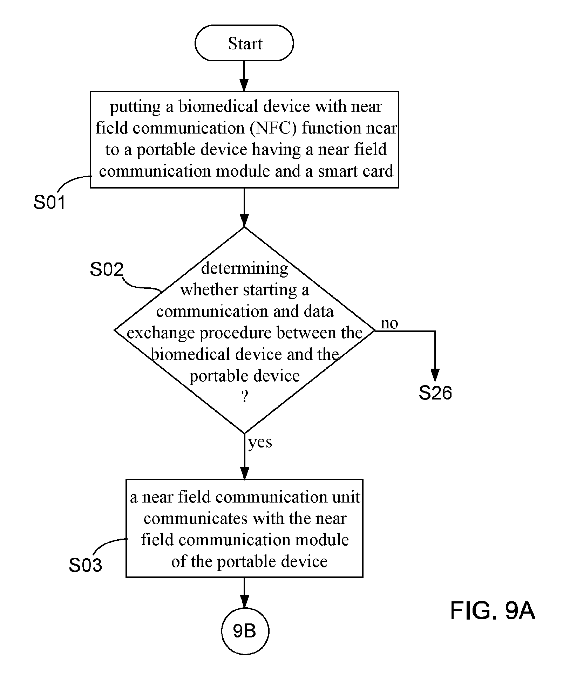

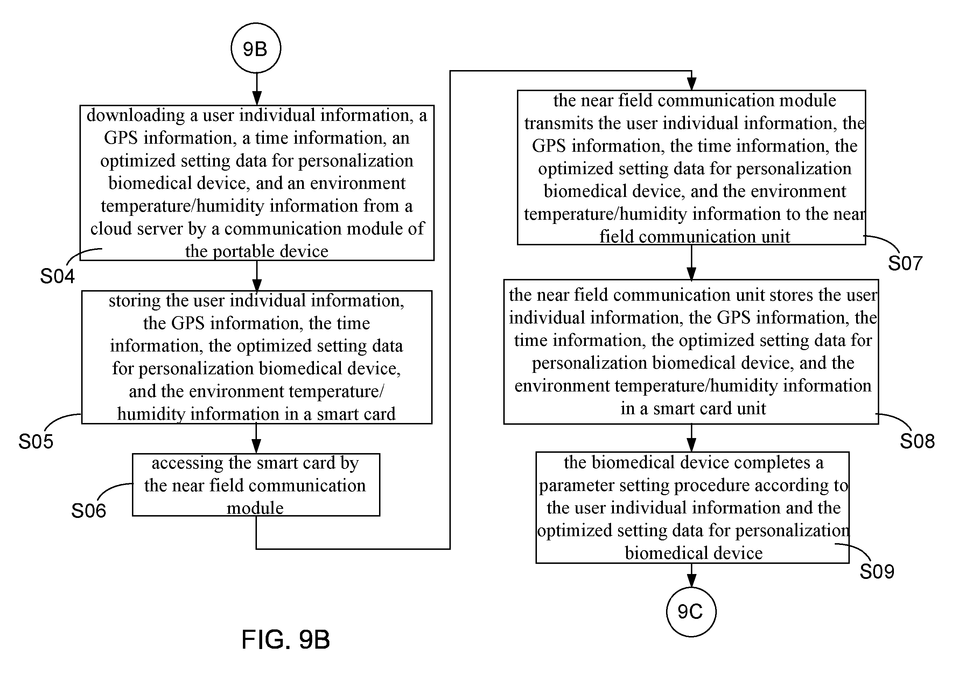

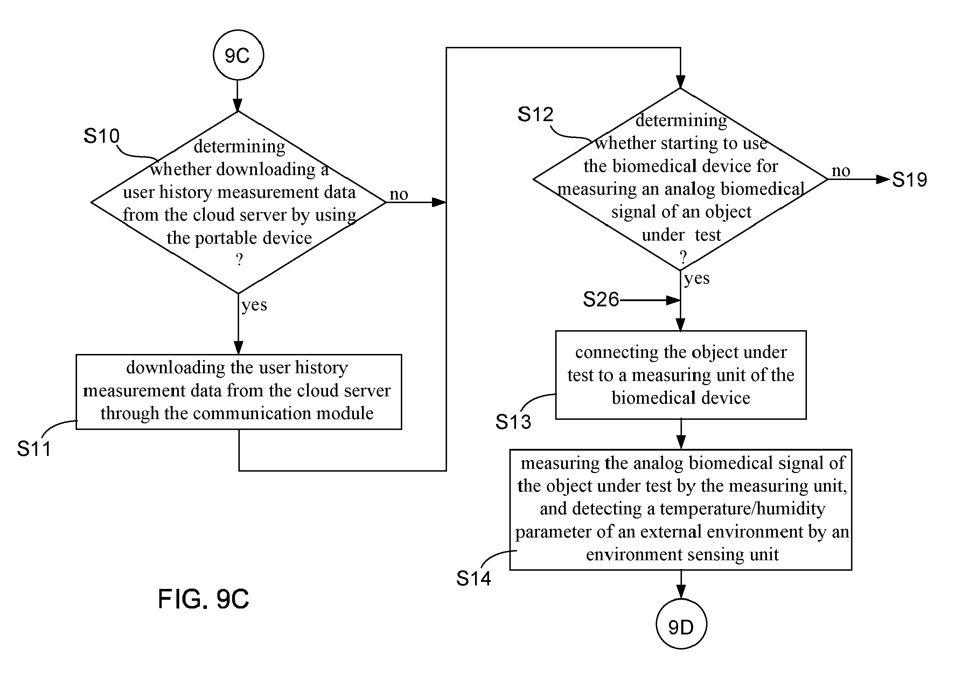

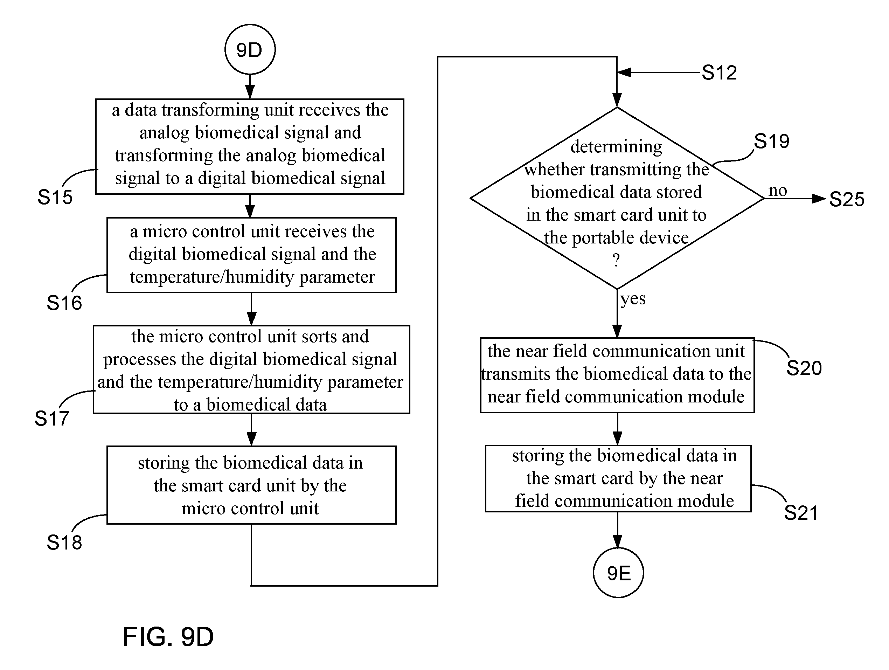

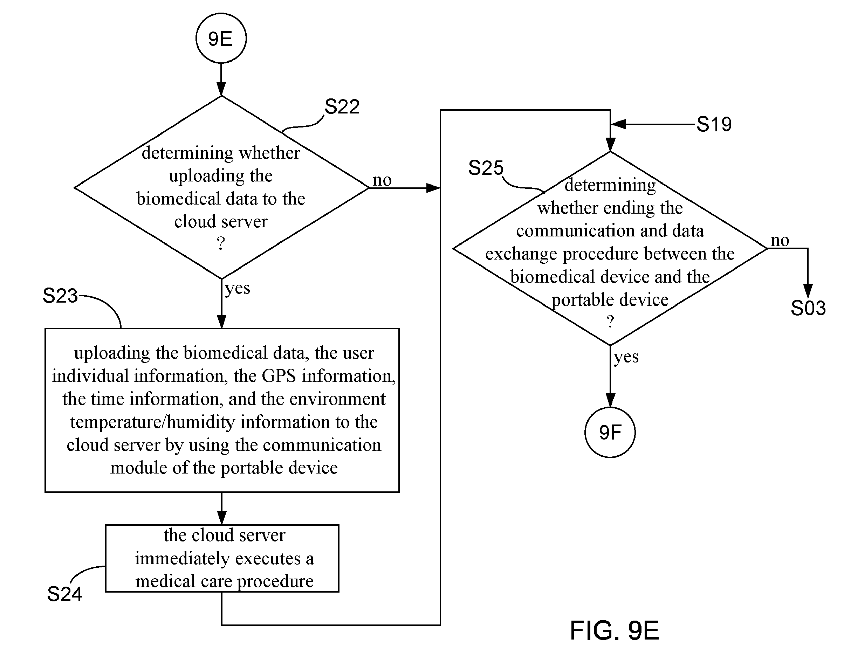

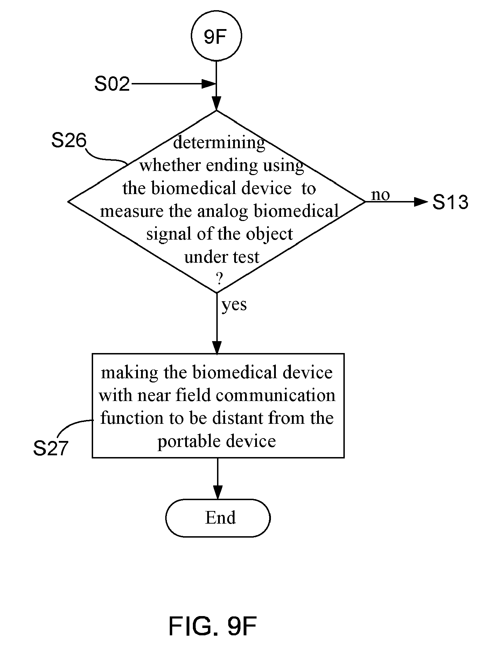

Thus, through above descriptions, all the practicable frameworks and the relative functionalities of the biomedical device 1 with NFC function have been completely introduced and disclosed; Subsequently, a method used by a biomedical device with near field communication (NFC) function for user identification, biomedical data measurement, biomedical data upload/download, biomedical data management, and remote medical care will be introduced and disclosed in follows. Please refer to FIGs. 9A to 9F which illustrate the flow chart of the method used by the biomedical device with near field communication (NFC) function for user identification, biomedical data measurement, biomedical data upload/download, biomedical data management, and remote medical care according to the present invention. As shown in FIG. 9A- FIG. 9E, the method used by the biomedical device with near field communication (NFC) function for user identification, biomedical data measurement, biomedical data upload/download, biomedical data management, and remote medical care includes the steps of:

Firstly, the flow proceeds step (S01), putting a biomedical device 1 with near field communication (NFC) function near to a portable device 2 having a near field communication module 21 and a smart card 22. After the biomedical device 1 is near to the portable device 2, the flow proceeds to step (S02), determining whether starting a communication and data exchange procedure between the biomedical device 1 and the portable device 2, if yes, proceeding to step (S03), a near field communication unit 18 communicates with the near field communication module 21 of the portable device 2. The flow continuously proceeds to step (S04), downloading a user individual information, a GPS information, a time information, an optimized setting data for personalization biomedical device, and an environment temperature/humidity information from a cloud server by a communication module 23 of the portable device 2. Then, the flow proceeds to step (S05), storing the user individual information, the GPS information, the time information, the optimized setting data for personalization biomedical device, and the environment temperature/humidity information in a smart card 22.

After the step (S05) is completed, the flow proceeds to step (S06), accessing the smart card 22 by the near field communication module 21, and subsequently proceeds to step (S07), the near field communication module 21 transmits the user individual information, the GPS information, the time information, the optimized setting data for personalization biomedical device, and the environment temperature/humidity information to the near field communication unit 18. The flow next proceeds to step (S08), the near field communication unit 18 stores the user individual information, the GPS information, the time information, the optimized setting data for personalization biomedical device, and the environment temperature/humidity information in a smart card unit 16.

When the step (S08) is finished, subsequently, the flow proceeds to step (S09), the biomedical device 1 completes a parameter setting procedure according to the user individual information and the optimized setting data for personalization biomedical device. Then, the flow proceeds to step (S10), determining whether downloading a user history measurement data from the cloud server by using the portable device 2, if yes, proceeding to step (S11), downloading the user history measurement data from the cloud server through the communication module 23, and continuously proceeds to step (S12); Moreover, when the determining result of the step (S10) is "no", the flow is directly proceeded to the step (S12), determining whether starting to use the biomedical device 1 for measuring an analog biomedical signal of an object under test 3.

In the determining step of the step (S12), if the determining result is "yes", the flow is proceeded to step (S13), connecting the object under test 3 to a measuring unit 11 of the biomedical device 1; then the flow subsequently proceeds to step (S14), measuring the analog biomedical signal of the object under test 3 by the measuring unit 11, and detecting a temperature/humidity parameter of an external environment by an environment sensing unit 12. Next, the flow proceeds to step (S15), a data transforming unit 13 receives the analog biomedical signal and transforming the analog biomedical signal to a digital biomedical signal. After the analog biomedical signal is transformed to the digital biomedical signal, the flow proceeds to step (S16), a micro control unit 14 receives the digital biomedical signal and the temperature/humidity parameter.

When the step (S16) is completed, the flow continuously proceeds to step (S17), the micro control unit 14 sorts and processes the digital biomedical signal and the temperature/humidity parameter to a biomedical data. Then, the flow proceeds to step (S 18), storing the biomedical data in the smart card unit 16 by the micro control unit 14, and proceeds to step (S19), determining whether transmitting the biomedical data stored in the smart card unit 16 to the portable device 2, if yes, proceeding to step (S20), the near field communication unit 18 transmits the biomedical data to the near field communication module 21. After the near field communication module 21 receives the biomedical data, the flow proceeds to step (S21), storing the biomedical data in the smart card 22 by the near field communication module 21.

When the biomedical data is stored in the smart card 22, subsequently, the flow proceeds to step (S22), determining whether uploading the biomedical data to the cloud server, if yes, proceeding to step (S23), uploading the biomedical data, the user individual information, the GPS information, the time information, and the environment temperature/humidity information to the cloud server by using the communication module 23 of the portable device 2. Then, the flow proceeds to step (S24), the cloud server immediately executes a medical care procedure, and subsequently proceeds to step (S25), determining whether ending the communication and data exchange procedure between the biomedical device 1 and the portable device 2, if yes, proceeding to step (S26), determining whether ending using the biomedical device 1 to measure the analog biomedical signal of the object under test 3, if yes, proceeding to step (S27), making the biomedical device 1 with near field communication function to be distant from the portable device 2.

Moreover, in the above flow chart of the method, when the determining result of the step (S02) is "no", the flow is proceeded to the step (S26); in addition, when the determining result of the step (S12) is "no", it means the user not needs to use the biomedical device 1 to measure the analog biomedical signal of the object under test 3; meanwhile, the flow is proceeded to the step (S19) for inquiring the user whether transmitting the biomedical data stored in the smart card unit 16 to the portable device 2. Moreover, when the determining results of the step (S19) and the step (S22) are "no", the flow is proceeded to the step (S25); furthermore, when the determining result of the step (S25) is "no", the flow is proceeded back to the step (S03); finally, when the determining result of the step (S26) is "no", the flow is proceeded back to the step (S 13).

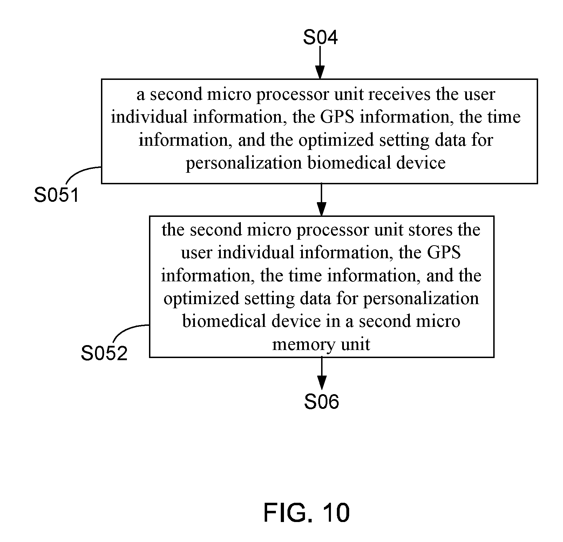

Besides, in the flow of the method used by the biomedical device with near field communication (NFC) function for user identification, biomedical data measurement, biomedical data upload/download, biomedical data management, and remote medical care, the step (S05), step (S06), step (S08), step (S18), step (S21), and step (S24) include at least two detailed steps. Please refer to FIG. 10, which illustrates a detailed flow chart of step (S05), as shown in FIG. 10, the step (S05) includes two detailed steps of: step (S051), a second micro processor unit 221 receives the user individual information, the GPS information, the time information, and the optimized setting data for personalization biomedical device; and step (S052), the second micro processor unit 221 stores the user individual information, the GPS information, the time information, and the optimized setting data for personalization biomedical device in a second micro memory unit 222.

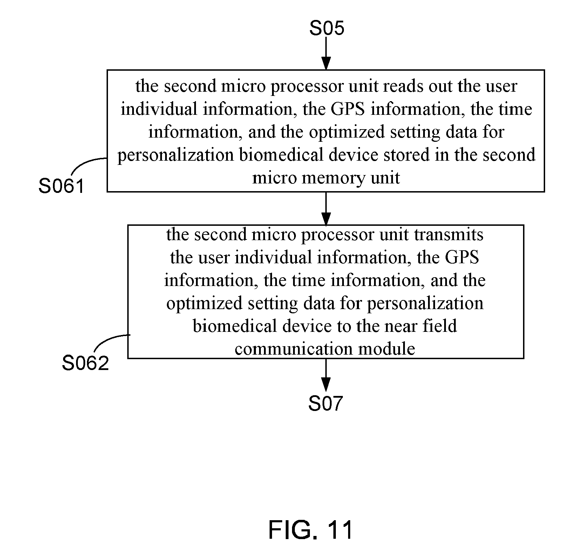

Moreover, please refer to FIG. 11, which illustrates the detailed flow chart of step (S06), as shown in FIG. 10, the step (S06) includes two detailed steps of: step (S061), the second micro processor unit 221 reads out the user individual information, the GPS information, the time information, and the optimized setting data for personalization biomedical device stored in the second micro memory unit 222; and step (S062), the second micro processor unit 221 transmits the user individual information, the GPS information, the time information, and the optimized setting data for personalization biomedical device to the near field communication module 21.

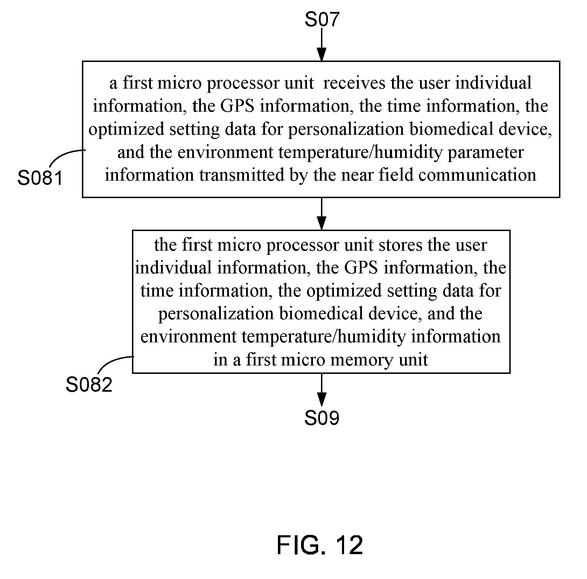

In addition, please refer to FIG. 12, which illustrates the detailed flow chart of step (S08), as shown in FIG. 12, the step (S08) includes two detailed steps of: step (S081), a first micro processor unit 161 receives the user individual information, the GPS information, the time information, the optimized setting data for personalization biomedical device, and the environment temperature/humidity parameter information transmitted by the near field communication 18; and step (S082), the first micro processor unit 161 stores the user individual information, the GPS information, the time information, the optimized setting data for personalization biomedical device, and the environment temperature/humidity information in a first micro memory unit 162.

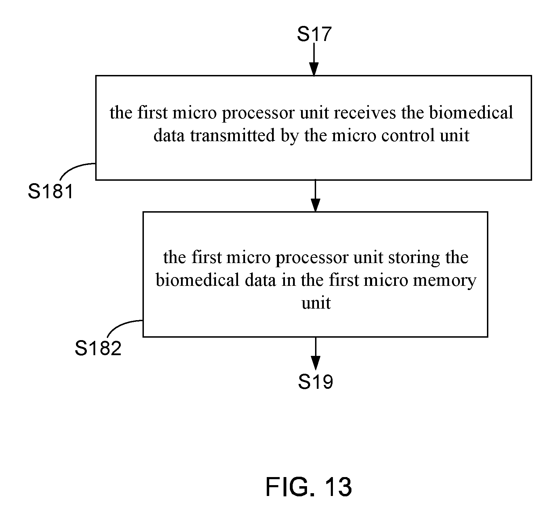

Furthermore, please refer to FIG. 13, which illustrates the detailed flow chart of step (S18), as shown in FIG. 13, the step (S18) includes two detailed steps of: step (S181), the first micro processor unit 161 receives the biomedical data transmitted by the micro control unit 14; and step (S182), the first micro processor unit 161 storing the biomedical data in the first micro memory unit 162.

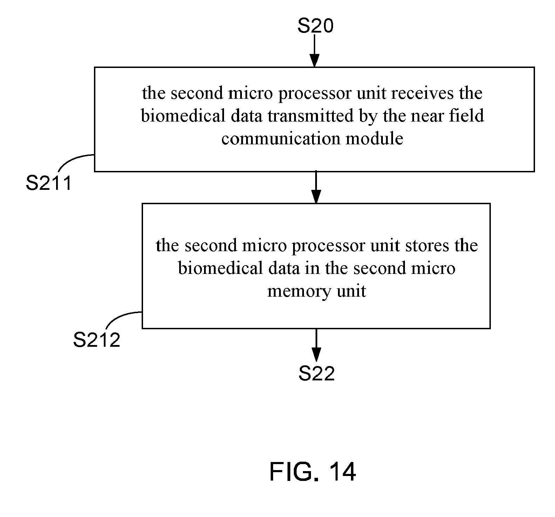

Please continuously refer to FIG. 14, which illustrates the detailed flow chart of the step (S21); similarly, as shown in FIG. 14, the step (S21) also includes two detailed steps of: step (S211), the second micro processor unit 221 receives the biomedical data transmitted by the near field communication module 21; and step (S212), the second micro processor unit 221 stores the biomedical data in the second micro memory unit 222.

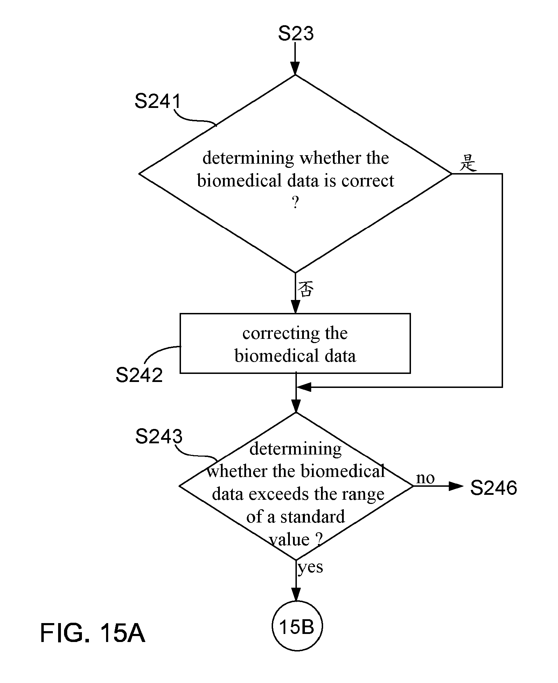

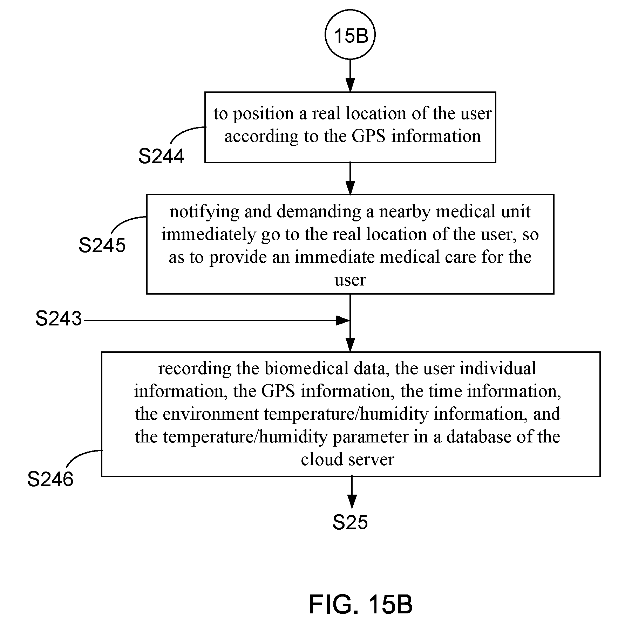

Moreover, in the method of the present invention, the step (S24) is the step that the cloud server executes the medical care procedure, so that, the step (S24) includes complicated detailed steps. Please refer to FIG. 15A and FIG. 15B, which illustrate the detailed flow chart of the step (S24), as shown in FIGs. 15A and 15B, the step (S24) includes the following detailed steps of:

Firstly, the flow proceeds to step (S241), determining whether the biomedical data is correct, if yes, proceeding to step (S243), determining whether the biomedical data exceeds the range of a standard value, if yes, proceeding to step (S244), to position a real location of the user according to the GPS information; and subsequently, the flow proceeds to step (S245), notifying and demanding a nearby medical unit immediately go to the real location of the user, so as to provide an immediate medical care for the user. Next, the flow proceeds to step (S246), recording the biomedical data, the user individual information, the GPS information, the time information, the environment temperature/humidity information, and the temperature/humidity parameter in a database of the cloud server. Besides, when the determining result of the step (S241) is "no", the flow is proceeded to step (S242), correcting the biomedical data and continuously proceeds to the next flow step; moreover, when the determining result of the step (S243) is "no", the flow is proceeded to step (S246).

Thus, through the above descriptions, biomedical device with near field communication (NFC) function and a method thereof for user identification, biomedical data measurement, biomedical data upload/download, biomedical data management, and remote medical care of the present invention have been disclosed completely and clearly in the above description. In summary, the present invention has the following advantages:

1. In the present invention, a smart unit and a near field communication unit are integrated in a biomedical device, for example, a blood glucose meter, therefore, after the blood glucose meter is used to measure and calculate a biomedical data of an object under test, the biomedical data would be stored in the smart card unit; and furthermore, through the near field communication unit, the biomedical data can be transmitted to a portable device, for instance, a smart phone, such that the biomedical data is able to be uploaded to a cloud server via the portable device; and then, the cloud server would record the biomedical data and provide the user an immediately medical care.

2. Inheriting to above point 1, what the feature of the present invention is, when the biomedical device with NFC function is near to the portable device, the user is able to download a user individual information and an optimized setting data for personalization biomedical device from the cloud server through the portable device, and transmit the user individual information and the optimized setting data for personalization biomedical device to the biomedical device by way of near field communication, such that the biomedical device can complete a basic parameter setting procedure according to the optimized setting data for personalization biomedical device.

3. Inheriting to above point 2, by means of identify the user individual information, the biomedical device is aware of the user identity and able to automatically execute the optimized parameter setting procedure; thus, multi men are allowed for using the identical biomedical device and the situation about misrecording and misjudging of data would not be occurred, and such biomedical device using way is very smart and economical.

4. Inheriting to above point 1, when the biomedical device is communicated with the portable device, the biomedical device can download a history data recorded in the database from the cloud server for being a comparison data.

5. The present invention further provides a method used by a biomedical device with near field communication function for user identification, biomedical data measurement, biomedical data upload/download, biomedical data management, and remote medical care, through the method, a communication and data exchange procedure between the biomedical device and a portable device can be finished, moreover, the biomedical data, the user individual information, the GPS information, the time information, and the environment temperature/humidity information are able to be uploaded to a cloud server through a communication module of the portable device, so that the cloud server is able to judge the data and then to immediately execute a medical care procedure.

6. Currently, everyone has at least one portable device, such as smart phone or tablet PC, and both the smart phone and the tablet PC have a long-distance communication function; Therefore, differing from the conventional biomedical device with the long-distance communication function, the main spirit of the present invention is that: to design a biomedical device with simple framework and economic cost, and the biomedical device is capable of communicating with the portable device, so as to further communicate with the cloud server through the portable device, such that the cloud server is able to receive the measured biomedical data transmitted by the biomedical device; furthermore, the cloud server can provide the immediately medical care to the user according to the received biomedical data thereof.

The above description is made on embodiments of the present invention. However, the embodiments are not intended to limit scope of the present invention, and all equivalent implementations or alterations within the spirit of the present invention still fall within the scope of the present invention.

## Claims

1. A biomedical device with near field communication (NFC) function, capable of communicating with a portable device having at least one near field communication module and a smart card, and able to exchange data with portable device by way of near field communication, the biomedical device comprising:
a measuring unit, being used for connecting an object under test, so as to measure an analog biomedical signal of the object under test;
**an environment sensing unit, capable of simultaneously detecting a** temperature/humidity parameter of the external environment when the measuring unit measures the analog biomedical signal;
a data transforming unit, being coupled to the measuring unit for receiving the analog biomedical signal, so as to transform the analog biomedical signal to a digital biomedical signal;
a micro control unit, being coupled to the environment sensing unit and the data transforming unit, and adopted for simultaneously receiving the digital biomedical signal and the temperature/humidity parameter in order to sort and process the digital biomedical signal and the temperature/humidity parameter to a biomedical data;
a memory unit, being coupled to the micro control unit for being one storage media;
a smart card unit, being coupled to the micro control unit for being one storage media;
a display unit, being coupled to the micro control unit and capable of receiving the biomedical data outputted by the micro control unit, so as to show a biomedical value; and
a near field communication (NFC) unit, being coupled to the smart card unit, wherein the near field communication unit is used for accessing the smart card unit, moreover, the near field communication unit being able to transmit the biomedical data stored in the smart card unit to the near field communication module of the portable device;
wherein the user must put the near field communication unit near to the near field communication module of the portable device before the object under test is connected to the measuring unit, such that the near field communication module can communicate with the near field communication unit, and the near field communication module can transmit a user individual information, a GPS information, a time information, an optimized setting data for personalization biomedical device, and an environment temperature/humidity information stored in the smart card to the near field communication unit; furthermore, the near field communication unit storing the user individual information, the GPS information, the time information, the optimized **setting data for personalization biomedical device, and the environment** temperature/humidity information in the smart card unit 16, so as to complete a user identification procedure and a parameter setting procedure.

2. The biomedical device with near field communication function of Claim 1, further comprising a power unit, used for providing power to the measuring unit, the environment sensing unit, the data transforming unit, the micro control unit, the display unit, and the near field communication unit.

3. The biomedical device with near field communication function of Claim 1, wherein the smart card unit further comprises:
a first micro processor unit, being coupled to the micro control unit and the near field communication unit for receiving the biomedical data, the user individual information,
**the GPS information, the time information, the optimized setting data** for personalization biomedical device, and the environment temperature/humidity information; and
a first micro memory unit, being coupled to the first micro processor unit, wherein the first micro processor unit stores the user individual information, the GPS information, the time information, the optimized setting data for personalization biomedical device, and the environment temperature/humidity information in the first micro memory unit after the biomedical data is received.

4. The biomedical device with near field communication function of Claim 1, wherein the smart card further comprises:
a second micro processor unit, being coupled to the near field communication module; and
a second micro memory unit, being coupled to the second micro processor unit, wherein the second micro processor unit is able to read out the user individual information, the GPS information, the time information, the optimized setting data for personalization biomedical device, and the environment temperature/humidity information stored in the second micro memory unit, and able to store the received biomedical data thereof in the second micro memory unit.

5. The biomedical device with near field communication function of Claim 1, wherein after the near field communication unit stores the user individual information in the smart card unit and the user identification procedure and the parameter setting procedure are finished, the user can further connect the object under test to the measuring unit for measuring the analog biomedical signal.

6. The biomedical device with near field communication function of Claim 3, wherein after the near field communication unit stores the user individual information in the smart card unit and the user identification procedure and the parameter setting procedure are finished, the near field communication unit can further transmit the biomedical data stored in the first micro memory unit to the near field communication module through the micro processor unit.

7. The biomedical device with near field communication function of Claim 4, wherein after the near field communication module receives the biomedical data transmitted by the near field communication unit, the second micro processor unit would store the biomedical data in the second micro memory unit, moreover, by way of a communication module of the portable device, the biomedical data, the user individual information, the GPS information, the time information, the optimized setting data for personalization biomedical device, and the environment temperature/humidity information may be uploaded to a cloud server with at least one database, so that a medical care procedure can be executed by way of the cloud server.

8. The biomedical device with near field communication function of Claim 1, wherein the measuring unit is selected from the group consisting of: a measuring device for blood glucose strip, a measuring device for body fat, a measuring device for blood pressure, and a measuring device for electrocardiography.

9. The biomedical device with near field communication function of Claim 1, wherein the object under test is selected from the group consisting of: a blood glucose strip and a man body.

10. The biomedical device with near field communication function of Claim 1, wherein the wherein the portable device is selected from the group consisting of: a smart phone, a PDA, a portable game console, a digital photo frame, an iPAD, a notebook, and a tablet PC.

11. A method used by a biomedical device with near field communication (NFC) function for user identification, biomedical data measurement, biomedical data upload/download, biomedical data management, and remote medical care, comprising the steps of:
(1) putting a biomedical device with near field communication (NFC) function near to a portable device having at least one near field communication module and a smart card;
(2) determining whether starting a communication and data exchange procedure between the biomedical device and the portable device, if yes, proceeding to step (3), otherwise, proceeding to step (21);
(3) a near field communication unit communicating with the near field communication module of the portable device;
(4) downloading a user individual information, a GPS information, a time information, an optimized setting data for personalization biomedical device, and an environment temperature/humidity information from a cloud server by a communication module of the portable device;
(5) storing the user individual information, the GPS information, the time information, the optimized setting data for personalization biomedical device, and the environment temperature/humidity information in a smart card;
(6) accessing the smart card by the near field communication module;
(7) the near field communication module transmitting the user individual information, the **GPS information, the time information, the optimized setting data** for personalization biomedical device, and the environment temperature/humidity information to the near field communication unit 18;
(8) the near field communication unit storing the user individual information, the GPS information, the time information, the optimized setting data for personalization biomedical device, and the environment temperature/humidity information in a smart card unit;
(9) the biomedical device completing a parameter setting procedure according to the user individual information and the optimized setting data for personalization biomedical device;
(10) determining whether downloading a user history measurement data from the cloud server by using the portable device, if yes, proceeding to step (11), otherwise, proceeding to step (12);
(11) downloading the user history measurement data from the cloud server through the communication module;
(12) determining whether starting to use the biomedical device for measuring an analog biomedical signal of an object under test, if yes, proceeding to step (13), otherwise, proceeding to step (19);
(13) connecting the object under test to a measuring unit of the biomedical device;
(14) measuring the analog biomedical signal of the object under test by the measuring unit, and detecting a temperature/humidity parameter of an external environment by an environment sensing unit;
(15) a data transforming unit receiving the analog biomedical signal and transforming the analog biomedical signal to a digital biomedical signal;
(16) **a micro control unit receiving the digital biomedical signal and the** temperature/humidity parameter;
(17) the micro control unit sorting and processing the digital biomedical signal and the temperature/humidity parameter to a biomedical data;
(18) storing the biomedical data in the smart card unit by the micro control unit;
(19) determining whether transmitting the biomedical data stored in the smart card unit to the portable device, if yes, proceeding to step (20), otherwise, proceeding to step (25);
(20) the near field communication unit transmitting the biomedical data to the near field communication module;
(21) storing the biomedical data in the smart card by the near field communication module;
(22) determining whether uploading the biomedical data to the cloud server, if yes, proceeding to step (23), otherwise, proceeding to step (25);
(23) uploading the biomedical data, the user individual information, the GPS information, the time information, and the environment temperature/humidity information to the cloud server by using the communication module of the portable device;
(24) the cloud server immediately executing a medical care procedure;
(25) determining whether ending the communication and data exchange procedure between the biomedical device 1 and the portable device, if yes, proceeding to step (26), otherwise, proceeding to step (3);
(26) determining whether ending using the biomedical device to measure the analog biomedical signal of the object under test, if yes, proceeding to step (27), otherwise, proceeding to step (13); and
(27) making the biomedical device with near field communication function to be distant from the portable device.

12. The method used by a biomedical device with near field communication function for user identification, biomedical data measurement, biomedical data upload/download, biomedical data management, and remote medical care of Claim 11, wherein the step (5) further comprises the detailed steps of:
(51) a second micro processor unit receiving the user individual information, the GPS information, the time information, and the optimized setting data for personalization biomedical device; and
(52) the second micro processor unit storing the user individual information, the GPS information, the time information, and the optimized setting data for personalization biomedical device in a second micro memory unit.

13. The method used by a biomedical device with near field communication function for user identification, biomedical data measurement, biomedical data upload/download, biomedical data management, and remote medical care of Claim 11, wherein the step (6) further comprises the detailed steps of:
(61) a second micro processor unit reading out the user individual information, the GPS information, the time information, and the optimized setting data for personalization biomedical device stored in a second micro memory unit; and
(62) the second micro processor unit transmitting the user individual information, the GPS information, the time information, and the optimized setting data for personalization biomedical device to the near field communication module.

14. The method used by a biomedical device with near field communication function for user identification, biomedical data measurement, biomedical data upload/download, biomedical data management, and remote medical care of Claim 11, wherein the step (8) further comprises the detailed steps of:
(81) a first micro processor unit receiving the user individual information, the GPS information, the time information, the optimized setting data for personalization biomedical device, and the environment temperature/humidity parameter information transmitted by the near field communication; and
(82) the first micro processor unit storing the user individual information, the GPS information, the time information, the optimized setting data for personalization biomedical device, and the environment temperature/humidity information in a first micro memory unit.

15. The method used by a biomedical device with near field communication function for user identification, biomedical data measurement, biomedical data upload/download, biomedical data management, and remote medical care of Claim 11, wherein the step (18) further comprises the detailed steps of:
(181) a first micro processor unit receiving the biomedical data transmitted by the micro control unit; and
(182) the first micro processor unit storing the biomedical data in a first micro memory unit.

16. The method used by a biomedical device with near field communication function for user identification, biomedical data measurement, biomedical data upload/download, biomedical data management, and remote medical care of Claim 11, wherein the step (21) further comprises the detailed steps of:
(211) a second micro processor unit receiving the biomedical data transmitted by the near field communication module; and
(212) the second micro processor unit storing the biomedical data in a second micro memory unit.

17. The method used by a biomedical device with near field communication function for user identification, biomedical data measurement, biomedical data upload/download, biomedical data management, and remote medical care of Claim 11, wherein the step (24) further comprises the detailed steps of:
(241) determining whether the biomedical data is correct, if yes, proceeding to step (243), otherwise, proceeding to step (242);
(242) correcting the biomedical data;
(243) determining whether the biomedical data exceeds the range of a standard value, if yes, proceeding to step (244), otherwise, proceeding to step (246);
(244) to position a real location of the user according to the GPS information;
(245) notifying and demanding a nearby medical unit immediately go to the real location of the user, so as to provide an immediate medical care for the user; and
(246) recording the biomedical data, the user individual information, the GPS information, the time information, the environment temperature/humidity information, and the temperature/humidity parameter in a database of the cloud server.

18. The method used by a biomedical device with near field communication function for user identification, biomedical data measurement, biomedical data upload/download, biomedical data management, and remote medical care of Claim 11, wherein the measuring unit is selected from the group consisting of: a measuring device for blood glucose strip, a measuring device for body fat, a measuring device for blood pressure, and a measuring device for electrocardiography.

19. The method used by a biomedical device with near field communication function for user identification, biomedical data measurement, biomedical data upload/download, biomedical data management, and remote medical care of Claim 11, wherein the object under test is selected from the group consisting of: a blood glucose strip and a man body.

20. The method used by a biomedical device with near field communication function for user identification, biomedical data measurement, biomedical data upload/download, biomedical data management, and remote medical care of Claim 11, wherein the wherein the portable device is selected from the group consisting of: a smart phone, a PDA, a portable game console, a digital photo frame, an iPAD, a notebook, and a tablet PC.
